# DEMANDE DE BREVET EUROPEEN

(11) **EP 1 321 098 A1**
(43) Date de publication de la demande: **25.06.2003**
(21) Numéro de dépôt: 02293159.6
(22) Date de dépôt: 19.12.2002
(51) Int. Cl.: A61B 5/103, A61B 5/00

(54) **Dispositif et procédé d'évaluation de la région du contour autour de l'oeil**

(30) Priorité: 21.12.2001 FR 0116787
(71) Demandeur: L'OREAL, 75008 Paris (FR)
(72) Inventeur: Kauffmann, Myriam, 69006 Lyon (FR); Bazin, Roland, 91570 Bièvres (FR)
(74) Mandataire: Tanty, François

(57) **Abrégé**

La présente invention concerne un procédé pour permettre d'évaluer la typologie de la région s'étendant autour de l'oeil humain, caractérisé par le fait qu'il comporte les étapes suivantes :
- acquérir au moins une donnée d'au moins une zone de ladite région, représentative d'au moins un pigment présent dans le circuit sanguin et/ou d'au moins un pigment externe au circuit sanguin,
- acquérir au moins une donnée concernant le relief d'au moins une zone de ladite région et/ou au moins une donnée concernant des propriétés mécaniques d'au moins une zone de ladite région,
- délivrer sur la base au moins des données précédemment acquises au moins une information concernant la typologie de ladite région.

## Description

La présente invention concerne les procédés et dispositifs pour l'évaluation du contour des yeux.

L'article *Study of Causal Factor of Dark Circles Around the Eyes,* IFSCC Magazine, Vol. 4, No. 4/2001 décrit l'utilisation d'un laser Doppler et d'un chromamètre pour effectuer des mesures dans la région du contour de l'oeil et tenter d'expliquer la cause des cernes.

Il existe un besoin pour évaluer la typologie de la région s'étendant autour de l'oeil humain, notamment les cernes, les poches, les rides du coin de l'oeil et les irrégularités de la peau et des paupières.

L'invention a pour objet, selon l'un de ses aspects, un procédé facilitant l'évaluation de la typologie de ladite région et comportant les étapes suivantes :
- permettre d'acquérir au moins une donnée d'au moins une zone de ladite région, associée à au moins un pigment présent dans le circuit sanguin et/ou à au moins un pigment externe au circuit sanguin,
- permettre d'acquérir au moins une donnée concernant le relief d'au moins une zone de ladite région et/ou au moins une donnée concernant au moins une propriété mécanique d'au moins une zone de ladite région,
- délivrer sur la base au moins des données précédemment acquises au moins une information concernant la typologie de ladite région.

Le pigment présent dans le circuit sanguin (tous types de vaisseaux sanguins) est par exemple l'hémoglobine sous sa forme oxydée ou non oxydée.

Le pigment présent hors du circuit sanguin est par exemple une mélanine, la bilirubine, l'hémosidérine.

Grâce à l'invention, il est possible d'évaluer plus facilement, voire plus précisément, la typologie de la région s'étendant autour de l'oeil humain, et l'invention permet notamment d'automatiser au moins en partie cette évaluation.

L'invention peut permettre de classer dans différentes catégories la région du contour de l'oeil d'une personne et de déterminer en fonction de ce classement les soins pouvant être apportés, notamment les soins non thérapeutiques.

L'invention peut permettre par exemple d'éviter les erreurs de prescription telles que l'administration à une personne ayant des cernes mélaniques d'un produit vasculaire qui sera sans effet sur les cernes, la couleur de ces derniers étant due à la coloration naturelle de la peau.

L'acquisition d'au moins une donnée associée à au moins un pigment présent dans le circuit sanguin et/ou à au moins un pigment externe au circuit sanguin peut comporter l'acquisition d'au moins une donnée concernant la couleur. Elle peut comporter en variante, ou additionnellement, l'acquisition d'au moins une température et/ou d'au moins une vitesse de circulation du sang.

Selon un aspect de l'invention, le procédé peut comporter en outre l'étape suivante :
- acquérir au moins une donnée relative aux circonstances d'apparition et/ou à l'évolution d'au moins une caractéristique de ladite région dans le temps. Il peut s'agir par exemple des résultats de mesures effectuées à différents moments ou de réponses à un questionnaire, ce questionnaire comportant par exemple des questions relatives aux circonstances de la survenue des cernes ou des poches et à leur réversibilité. Le questionnaire peut comporter encore des questions concernant l'existence d'une gêne éventuelle, par exemple une sensation de picotements, en cas d'allergie.

Selon un aspect de l'invention, le procédé comporte les étapes suivantes :
- acquérir au moins une donnée concernant la couleur d'au moins une zone de ladite région,
- acquérir au moins une donnée concernant le relief d'au moins une zone de ladite région,
- acquérir au moins une donnée concernant au moins une propriété mécanique d'au moins une zone de ladite région,
- délivrer sur la base au moins des données précédemment acquises au moins une information concernant la typologie de ladite région.

Les données acquises peuvent être comparées à des données prédéterminées et au moins une information concernant la typologie de ladite région peut être délivrée au vu du résultat de cette comparaison.

Selon un autre aspect de l'invention, le procédé peut comporter l'étape consistant à déterminer la transparence de la peau d'au moins une zone de ladite région. La facilité du passage de la lumière à travers la peau peut être en effet responsable au moins en partie de la visibilité des cernes, ces derniers pouvant être dus à la présence des vaisseaux sanguins sous-jacents.

On peut utiliser pour acquérir au moins une donnée concernant la couleur d'au moins une zone de ladite région au moins l'un des éléments de la liste suivante : une caméra vidéo, un scanner, un appareil photo numérique, un spectrocolorimètre, un colorimètre, un nuancier, un SCIASCOPE™ (tel que décrit dans WO 00/75637), un MEXAMETRE™ (commercialisé par la société COURAGE et KHAZAKA), une caméra thermique.

On peut éclairer ladite région, lors de l'acquisition de ladite au moins une donnée concernant la couleur d'au moins une zone de ladite région, avec une lumière visible, ultraviolette ou infrarouge.

On peut aussi acquérir des données concernant la couleur d'au moins une zone de ladite région en éclairant successivement ladite zone avec des éclairages différents.

On peut acquérir au moins une donnée concernant le relief d'au moins une zone de ladite région au moyen d'un élément choisi dans la liste suivante : système à projection de franges, système à vision stéréoscopique, système d'imagerie à ultrasons, lampe à fente.

On peut acquérir au moins une donnée concernant des propriétés mécaniques d'au moins une zone de ladite région au moyen d'un élément choisi dans la liste suivante : palpeur mécanique, dispositif à pincement, dispositif à torsion, dispositif à aspiration, dispositif à projection de liquide ou de gaz, tonomètre, dispositif pléthysmographique.

Selon un aspect de l'invention, on peut effectuer dans un premier intervalle de temps au moins une première acquisition d'au moins une donnée concernant la couleur d'au moins une zone de ladite région et/ou d'au moins une donnée concernant le relief d'au moins une zone de ladite région et/ou d'au moins une donnée concernant au moins une propriété mécanique d'au moins une zone de ladite région. On peut procéder ensuite dans un deuxième intervalle de temps succédant au premier, à au moins une deuxième acquisition d'au moins une donnée concernant la couleur d'au moins une zone de ladite région et/ou d'au moins une donnée concernant le relief d'au moins une zone de ladite région et/ou d'au moins une donnée concernant au moins une propriété mécanique d'au moins une zone de ladite région.

Notamment lorsque les zones examinées lors des première et deuxième acquisitions sont les mêmes, on peut comparer les résultats des première et deuxième acquisitions, et tirer des résultats de la comparaison au moins une information concernant la typologie de ladite région.

L'information délivrée sur la base des diverses acquisitions effectuées peut être une information concernant les cernes, et notamment une information permettant de classer les cernes dans une catégorie parmi un ensemble comportant plusieurs catégories. Un tel ensemble peut comporter par exemple au moins trois types de cernes, les différents types de cernes pouvant être les suivants, par exemple : cernes ethniques, faux cernes, cernes au moins partiellement réversibles.

L'information concernant la typologie de la région du contour de l'oeil peut également être une information concernant les poches, par exemple une information permettant de classer les poches dans une catégorie parmi un ensemble de plusieurs catégories. Un tel ensemble peut comporter au moins quatre catégories, par exemple les catégories suivantes : poches creuses dues au relâchement des tissus, poches pleines dues à la protrusion des loges graisseuses, poches pleines dues à la ptose des tissus (par exemple le relâchement des muscles élévateurs), poches pleines dues au gonflement lymphoedémateux.

L'information concernant la typologie de la région du contour de l'oeil peut aussi être une information concernant les rides du coin de l'oeil, par exemple leur nombre, leur longueur, leur profondeur.

L'invention a encore pour objet, selon un autre de ses aspects, un procédé pour évaluer l'incidence sur l'aspect de la région autour de l'oeil de l'application d'un produit, notamment cosmétique, ou pour déterminer l'efficacité d'un traitement, dans lequel :
- on procède à une première acquisition de données associées à au moins un pigment intérieur et/ou extérieur au circuit sanguin, notamment au moins une donnée concernant la couleur, et de données concernant le relief et/ou les propriétés mécaniques d'au moins une zone de la région autour de l'oeil avant l'application du produit et/ou un traitement, notamment cosmétique ou chirurgical,
- on applique le produit et/ou l'on effectue le traitement,
- on procède ensuite à une deuxième acquisition de données associées audit au moins un pigment intérieur et/ou extérieur au circuit sanguin, notamment au moins une donnée concernant la couleur, et de données et concernant le relief et/ou les propriétés mécaniques d'au moins une zone de la région autour de l'oeil,
- on détermine en comparant les résultats des première et deuxième acquisitions l'incidence sur l'aspect de la région autour de l'oeil de l'application du produit et/ou l'efficacité du traitement.

Pour comprendre ce que l'on entend par « produit cosmétique », on pourra se référer à la Directive 76/768/CEE telle que modifiée par la Directive 96/35/CEE du 14 juin 1993. Par traitement cosmétique, on entend tout traitement non thérapeutique au moyen d'un produit cosmétique tel que défini ci-dessus.

La présente invention a encore pour objet, selon un autre de ses aspects, un dispositif comportant des moyens d'acquisition permettant d'acquérir :
- au moins une donnée associée à au moins un pigment présent dans le circuit sanguin et/ou à au moins un pigment externe au circuit sanguin, notamment une donnée concernant la couleur, d'au moins une zone de ladite région,
   et permettant d'acquérir également :

- au moins une donnée concernant le relief d'au moins une zone de ladite région et/ou,
- au moins une donnée concernant au moins une propriété mécanique d'au moins une zone de ladite région.

Par «dispositif», il faut comprendre au sens de la présente invention un appareil de construction unitaire, pouvant éventuellement être tenu d'une main, ou un ensemble d'appareils.

Selon un aspect de l'invention, le dispositif comporte en outre des moyens de traitement pour comparer les données acquises à des données mémorisées et générer au moins une information concernant la typologie de la région observée en fonction du résultat de cette comparaison. L'information générée peut être une valeur numérique ou une information non numérique.

Le dispositif peut être agencé pour permettre l'acquisition de données relatives à l'évolution dans le temps d'au moins une caractéristique de la région en question.

Le dispositif peut comporter des moyens d'acquisition colorimétriques comprenant au moins l'un des éléments de la liste suivante : une caméra vidéo, un scanner, un appareil photo numérique, un spectrocolorimètre, un colorimètre, un nuancier, une caméra thermique.

Le dispositif peut comporter des moyens d'éclairage permettant d'éclairer ladite région avec une lumière visible, ultraviolette ou infrarouge.

Le dispositif peut comporter des moyens permettant d'effectuer des acquisitions colorimétriques sous des éclairages différents ou par transparence.

Le dispositif peut comporter des moyens d'acquisition du relief comportant au moins un élément choisi dans la liste suivante : système à projection de franges, système à vision stéréoscopique, système d'imagerie à ultrasons, lampe à fente.

Le dispositif peut comporter des moyens d'acquisition d'au moins une propriété mécanique comportant au moins un élément choisi dans la liste suivante : palpeur mécanique, dispositif à pincement, dispositif à torsion, dispositif à aspiration, dispositif à projection de liquide ou de gaz, dispositif tonométrique, dispositif pléthysmographique.

Le dispositif peut comporter des moyens de stockage permettant de stocker des données acquises à différents instants.

Le dispositif peut comporter un cache agencé pour s'appliquer par la totalité de son pourtour contre une région du contour de l'oeil.

L'évaluation de la région du contour de l'oeil peut être effectuée dans un but non thérapeutique, purement esthétique.

L'invention a encore pour objet, selon l'un de ses aspects, un procédé pour diagnostiquer au moins un état de la région du contour de l'oeil, comportant les étapes suivantes :
- permettre d'acquérir au moins une donnée d'au moins une zone de ladite région, associée à au moins un pigment présent dans le circuit sanguin et/ou à au moins un pigment externe au circuit sanguin,
- permettre d'acquérir au moins une donnée concernant le relief d'au moins une zone de ladite région et/ou au moins une donnée concernant au moins une propriété mécanique d'au moins une zone de ladite région,
- délivrer un diagnostic, notamment un diagnostic non médical, sur la base au moins des données précédemment acquises.

L'invention a encore pour objet, selon un autre de ses aspects, un procédé pour prescrire un traitement, notamment un traitement cosmétique, comportant les étapes suivantes :
- permettre d'acquérir au moins une donnée d'au moins une zone de ladite région, associée à au moins un pigment présent dans le circuit sanguin et/ou à au moins un pigment externe au circuit sanguin,
- permettre d'acquérir au moins une donnée concernant le relief d'au moins une zone de ladite région et/ou au moins une donnée concernant au moins une propriété mécanique d'au moins une zone de ladite région,
- prescrire un traitement sur la base au moins des données précédemment acquises.

Le produit prescrit est par exemple un produit comportant un actif dépigmentant (par exemple l'acide kojique), un filtre UV, un produit anti-allergique, un anti-inflammatoire.

L'invention a encore pour objet, selon un autre de ses aspects, un kit permettant à un individu de s'auto-diagnostiquer, comportant :
- des moyens (par exemple un miroir) permettant d'acquérir au moins une donnée d'au moins une zone de ladite région, associée à au moins un pigment présent dans le circuit sanguin et/ou à au moins un pigment externe au circuit sanguin,
- des moyens (par exemple un accessoire spécifique) permettant d'acquérir au moins une donnée concernant le relief d'au moins une zone de ladite région et/ou au moins une donnée concernant au moins une propriété mécanique d'au moins une zone de ladite région,
- des moyens (par exemple un atlas) permettant de délivrer un diagnostic, notamment un diagnostic non médical, sur la base au moins des données précédemment acquises.

L'invention a encore pour objet, selon un autre de ses aspects, un procédé de fabrication d'un produit, notamment cosmétique, comportant les étapes suivantes :
- permettre d'acquérir au moins une donnée d'au moins une zone de ladite région, associée à au moins un pigment présent dans le circuit sanguin et/ou à au moins un pigment externe au circuit sanguin,
- permettre d'acquérir au moins une donnée concernant le relief d'au moins une zone de ladite région et/ou au moins une donnée concernant au moins une propriété mécanique d'au moins une zone de ladite région,
- fabriquer un produit, notamment cosmétique, au moins sur la base des données précédemment acquises.

L'invention a encore pour objet un procédé pour générer un panel d'utilisateurs potentiels d'un produit, notamment cosmétique, comportant les étapes suivantes :
- pour chaque individu d'un groupe, acquérir :

- au moins une donnée d'au moins une zone de ladite région, associée à au moins un pigment présent dans le circuit sanguin et/ou à au moins un pigment externe au circuit sanguin,
- au moins une donnée concernant le relief d'au moins une zone de ladite région et/ou au moins une donnée concernant au moins une propriété mécanique d'au moins une zone de ladite région,
- sélectionner parmi les individus de ce groupe ceux dont les données précédemment acquises répondent à au moins un critère prédéterminé.

Le procédé ci-dessus peut comporter en outre les étapes suivantes :
i) générer un panel,
ii) fabriquer un produit, notamment un produit cosmétique,
iii) appliquer ou administrer le produit à des individus du panel,
iv) effectuer une nouvelle évaluation,
v) comparer les résultats de la nouvelle évaluation avec ceux de la ou des évaluations précédentes, en vue de déterminer l'efficacité du produit,
vi) si l'efficacité du produit est jugée insuffisante, modifier la formulation du produit et/ou sa posologie, et recommencer les étapes iii), iv) et v) jusqu'à l'obtention d'une efficacité jugée suffisante.

L'invention a encore pour objet, selon un autre de ses aspects, un procédé pour déterminer l'efficacité d'un traitement, notamment un traitement cosmétique, comportant les étapes suivantes :
- effectuer une première évaluation de la région du contour de l'oeil,
- effectuer un traitement,
- effectuer une nouvelle évaluation
- comparer les résultats des évaluations et en tirer une information utile sur l'efficacité du traitement.

L'invention pourra être mieux comprise à la lecture de la description qui va suivre, d'exemples non limitatifs de mise en oeuvre, et à l'examen du dessin annexé, qui fait partie intégrante de la description, et sur lequel :
- la figure 1 représente de manière très schématique un exemple de dispositif pouvant être utilisé pour évaluer la région du contour de l'oeil,
- la figure 2 est une vue schématique à échelle agrandie du dispositif de la figure 1, faisant apparaître différents éléments constitutifs de ce dispositif,
- la figure 3 est un exemple de dispositif pouvant être utilisé pour effectuer une mesure de la couleur et/ou du relief de la région du contour de l'oeil,
- la figure 4 illustre le positionnement du visage dans l'ouverture de la sphère du dispositif de la figure 3,
- la figure 5 donne un exemple de localisation des cernes mélaniques,
- la figure 6 donne un exemple de localisation de cernes autres que mélaniques,
- la figure 7 donne un exemple de localisation d'une poche,
- la figure 8 est un exemple de détermination d'une caractéristique de la typologie du contour de l'oeil au moyen d'une séquence d'images,
- la figure 9 est un schéma en blocs illustrant un aspect d'un exemple de procédé selon l'invention,
- la figure 10 illustre la transmission de données par réseau informatique, en vue par exemple d'évaluer à distance les données acquises, et
- la figure 11 représente un exemple de dispositif permettant de délivrer un produit en fonction du résultat d'une évaluation du contour de l'oeil.

L'évaluation de la région du contour de l'oeil peut s'effectuer conformément à l'invention au moyen d'un dispositif de construction unitaire ou d'un dispositif constitué par un ensemble d'appareils séparés. L'évaluation peut s'effectuer l'oeil étant ouvert, fermé, ou successivement fermé puis ouvert ou inversement. L'évaluation peut encore s'effectuer le visage immobile et détendu ou souriant et contracté

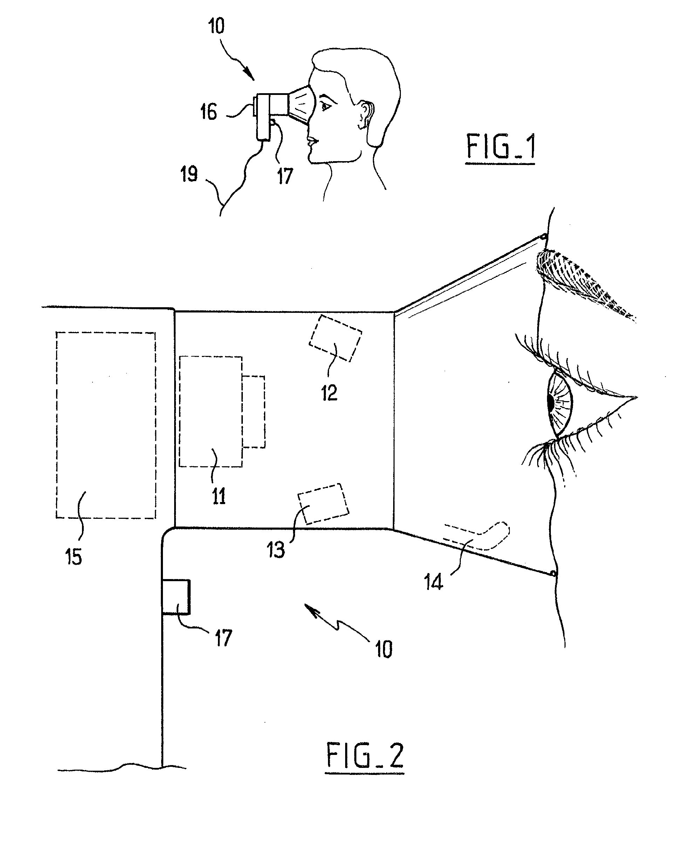

A titre d'exemple de construction unitaire, on a représenté sur les figures 1 et 2 un appareil 10 intégrant un certain nombre d'éléments permettant d'acquérir des données relatives par exemple à la couleur, ou la transparence, au relief et/ou aux propriétés mécaniques d'au moins une zone de la région du contour de l'oeil.

L'appareil 10 peut comporter par exemple une caméra 11 et au moins un dispositif d'éclairage 12.

La caméra 11 est par exemple du type CCD et l'image acquise peut être traitée afin de déterminer la couleur de chaque pixel et délimiter les zones ayant sensiblement la même couleur, afin par exemple d'évaluer l'étendue et/ou la localisation de cernes dus à un ou plusieurs pigments prédéterminés, par exemple. Le contour des cernes dus aux pigments circulant avec le sang peut par exemple être déterminé en détectant les zones les plus rouges de l'image acquise au moyen de la caméra 11. Cette dernière peut avoir été calibrée au préalable en plaçant une échelle de couleurs de référence dans son champ.

L'appareil 10 peut encore comporter par exemple au moins un dispositif d'évaluation du relief 13 permettant l'acquisition du relief d'au moins une zone de la région du contour de l'oeil, par exemple un dispositif à projection de franges.

L'appareil 10 peut encore comporter au moins un dispositif d'évaluation mécanique 14 permettant de déterminer, en combinaison éventuellement avec la caméra 11 et le dispositif d'évaluation du relief 13, au moins une propriété mécanique d'au moins une zone de la région du contour de l'oeil.

Le dispositif d'évaluation mécanique 14 peut comporter par exemple une buse permettant de projeter un jet d'air comprimé sur au moins une zone de la région du contour de l'oeil, afin de déformer cette zone, par exemple la paupière inférieure ou le dessus de la pommette.

Le dispositif d'éclairage 12 peut comporter par exemple au moins une source lumineuse telle qu'une lampe à incandescence ou à décharge ou une diode électroluminescente ou une diode laser, ou une combinaison de ces différentes sources. Le dispositif d'éclairage 12 peut comporter au moins une source lumineuse associée à un système de diffusion de la lumière, de manière à obtenir un éclairage sensiblement uniforme de la région du contour de l'oeil. Le dispositif d'éclairage 12 peut notamment comporter une pluralité de sources lumineuses réparties par exemple circonférentiellement. Le dispositif d'éclairage 12 peut également comporter un ou plusieurs guides de lumière, notamment des fibres optiques. Le dispositif d'éclairage 12 peut être agencé pour éclairer la région du contour de l'oeil avec une lumière présentant des caractéristiques spectrales bien définies, choisies par exemple pour faire ressortir au mieux un pigment particulier de la peau, par exemple la ferritine ou l'hémoglobine oxydée.

Le dispositif d'éclairage 12 peut également permettre d'illuminer successivement la peau avec des longueurs d'ondes différentes.

Ainsi, la peau peut être éclairée successivement avec une lumière visible, infrarouge et/ou ultraviolette, par exemple.

Le dispositif d'éclairage 12 peut être agencé, notamment orienté, de manière à permettre d'éliminer l'effet d'ombre des poches, pour un certain type de poches. En mesurant le degré d'atténuation de l'effet d'ombre sous un éclairage particulier, on peut tirer une information sur le caractère plus ou moins saillant des poches.

On peut utiliser comme moyens d'acquisition du relief d'autres moyens qu'un dispositif à projection de franges, par exemple un dispositif de vision stéréoscopique ou un dispositif télémétrique à ultrasons, cette liste n'étant pas limitative.

On peut utiliser comme moyens d'acquisition des propriétés mécaniques d'autres moyens qu'un dispositif à projection d'air comprimé, par exemple un dispositif opérant par pincement, torsion ou succion.

Le dispositif d'évaluation du relief peut être agencé pour coopérer avec le dispositif d'évaluation mécanique afin de mesurer par exemple la variation du relief sous l'effet d'une sollicitation mécanique. Ainsi, par exemple, la zone étudiée peut être sollicitée mécaniquement et la variation du relief qui en résulte mesurée optiquement pour déterminer la fermeté et/ou l'élasticité de la peau dans cette zone.

L'appareil 10 peut intégrer des moyens de traitement 15 des signaux émis par les différents éléments d'évaluation de la couleur, de la transparence, du relief et/ou des propriétés mécaniques, de tels moyens de traitement pouvant comporter un ou plusieurs micro-processeurs ou autres composants permettant d'effectuer les opérations nécessaires au traitement des données.

L'appareil 10 peut comporter en outre un afficheur 16, par exemple du coté opposé à l'ouverture dans laquelle est placée la région de l'oeil, ainsi qu'au moins un bouton de commande 17 permettant de commander son fonctionnement lors d'une séance d'acquisition de données.

L'afficheur 16 peut permettre d'afficher des informations numériques ou non, concernant la région observée, par exemple le type de cernes ou de poches.

L'appareil 10 peut être complètement autonome sur le plan énergétique ou être relié à une source d'énergie électrique.

Dans l'exemple décrit, l'appareil 10 comporte un cache 18 de forme sensiblement tronconique agencé pour s'appliquer par la totalité de son pourtour contre le visage autour de l'oeil, en épousant le relief du visage, par exemple celui de l'arcade sourcilière et d'une partie du nez.

Le pourtour du cache peut, avant son application contre le visage, être contenu en totalité dans un plan, auquel cas le cache peut comporter un joint en matériau élastiquement déformable qui définit le pourtour. La déformation du joint permet, lors de son application sur le visage autour de l'oeil, d'en épouser le relief.

Le cache peut en variante être dépourvu de joint et être réalisé lui-même en un matériau élastiquement déformable de manière à pouvoir épouser la forme du visage.

Le pourtour du cache peut encore n'être pas contenu dans un plan et présenter une forme adaptée lui permettant, sans déformation, d'épouser le relief du visage dans la région autour de l'oeil.

On ne sort pas du cadre de la présente invention lorsque l'appareil 10 n'intègre pas de moyens de traitement des informations délivrées par les différents éléments servant à l'acquisition des données et que le traitement des données est effectué par un ordinateur personnel ou un autre appareil, ou lorsque que le traitement des données est effectué à la fois par l'appareil servant à l'acquisition des données et par des moyens de traitement des données auquel l'appareil d'acquisition est relié et avec lesquels il peut échanger des informations, au moyen d'un câble ou d'une liaison sans fil, par exemple.

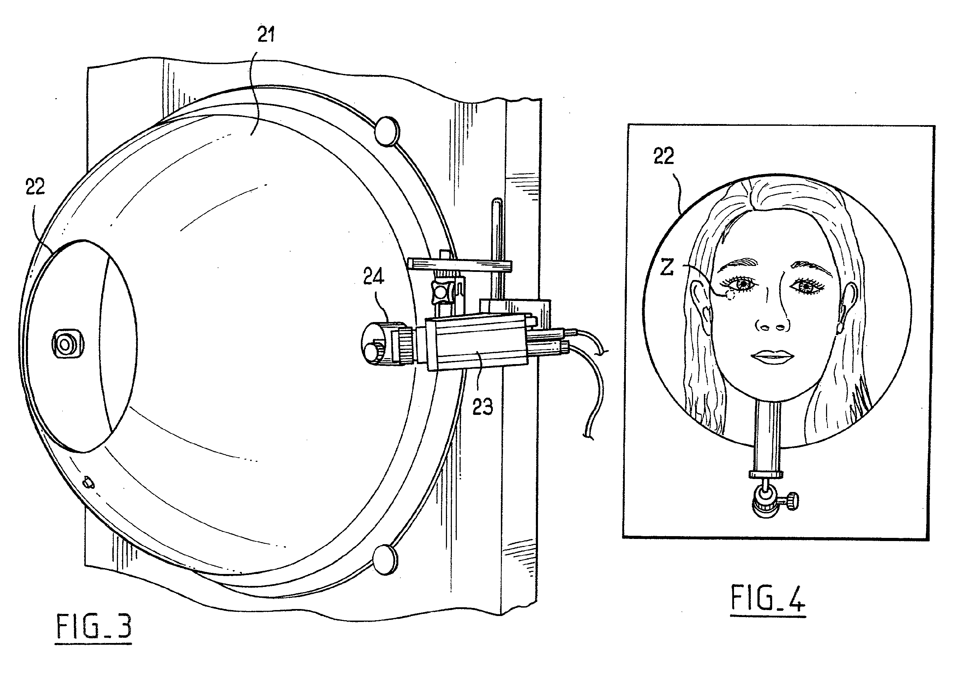

On a représenté partiellement sur les figures 3 et 4 un dispositif 20 permettant d'effectuer une acquisition de données colorimétriques et d'observer le relief de la région du contour de l'oeil.

Le dispositif 20 ne présente pas une construction unitaire, car il regroupe un ensemble d'appareils pouvant être utilisés de manière indépendante les uns des autres, non simultanément, et déplacés les uns par rapport aux autres.

Le dispositif 20 comporte, dans l'exemple illustré, une sphère 21 présentant une ouverture 22 dans laquelle une personne dont on souhaite évaluer la région du contour de l'oeil peut placer son visage et plusieurs caméras 23 permettant d'observer au moins une zone de la région du contour de l'oeil, par exemple la zone Z délimitée par un cercle en traits discontinus sur la figure 4.

Les caméras 23, au nombre de trois dans l'exemple illustré, permettent d'observer le visage de face et de côté, à travers des ouvertures 24 la sphère 21.

Les différentes caméras 23 peuvent permettre non seulement d'acquérir des informations concernant la couleur de la région autour de l'oeil mais également son relief, par stéréoscopie.

D'autres dispositifs pourraient encore être utilisés, par exemple celui décrit dans la demande WO 00/75637, dont le contenu est incorporé par référence.

Selon un aspect de l'invention, on utilise les différents moyens d'acquisition de données pour :
- acquérir au moins une donnée concernant la couleur d'au moins une zone de la région autour de l'oeil,
- acquérir au moins une donnée concernant le relief d'au moins une zone de ladite région et/ou au moins une donnée concernant des propriétés mécaniques d'au moins une zone de ladite région, et l'on délivre sur la base au moins des données précédemment acquises au moins une information concernant la typologie de ladite région.

L'invention permet notamment d'évaluer les cernes.

Les cernes peuvent avoir plusieurs causes. On peut ainsi distinguer au moins trois types de cernes, à savoir les cernes dits ethniques ou mélaniques, dus à la pigmentation naturelle de la peau, les faux cernes, c'est-à-dire les cernes dus à un effet d'ombre et de lumière à cause du relief de la région autour de l'oeil et les cernes dits vasculaires, liés par exemple à la maladie ou au manque de sommeil. Ces différentes catégories de cernes sont portées sur le tableau I ci-dessous.

**TABLEAU I**

| **Type cernes** | **Couleur** | **Localisation** | **Origine** | **Evolution** |
|---|---|---|---|---|
| Vasculaires | Violet, bleu, rouge | Paupière inférieure, angle avec la zone para nasale supérieure | Visibilité des éléments sanguins par transparence | Plus ou moins réversible |
| Ethniques | Brun, gris | Paupières inférieure et Supérieure | Mélanines | Peu réversible (s'assombrit après une exposition solaire) |
| Faux cernes | --- | Paupière inférieure | Effet d'ombre et de lumière | Selon l'évolution des poches |

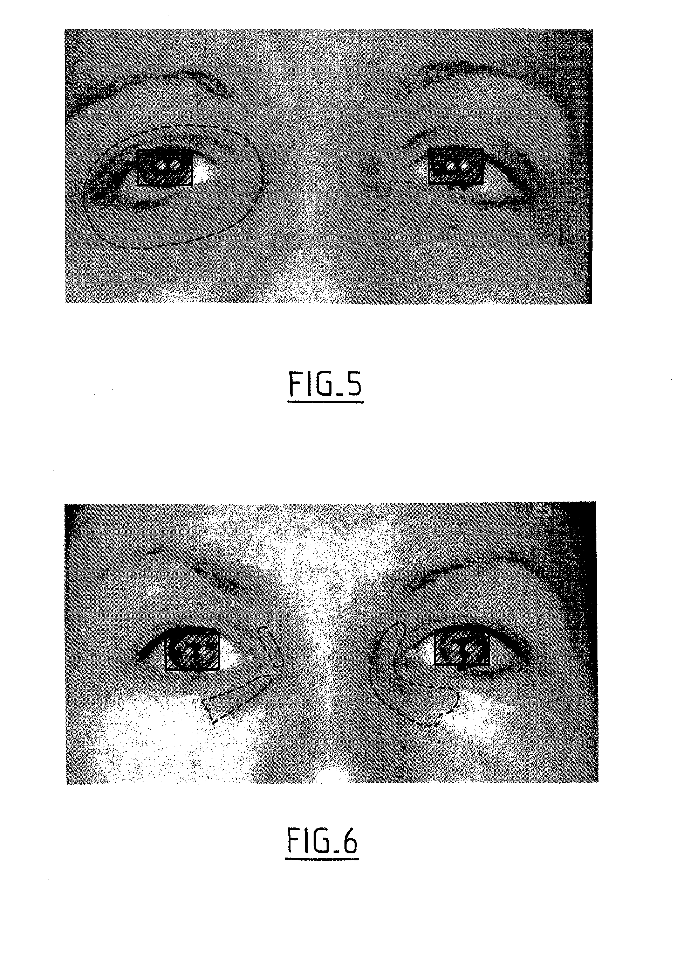

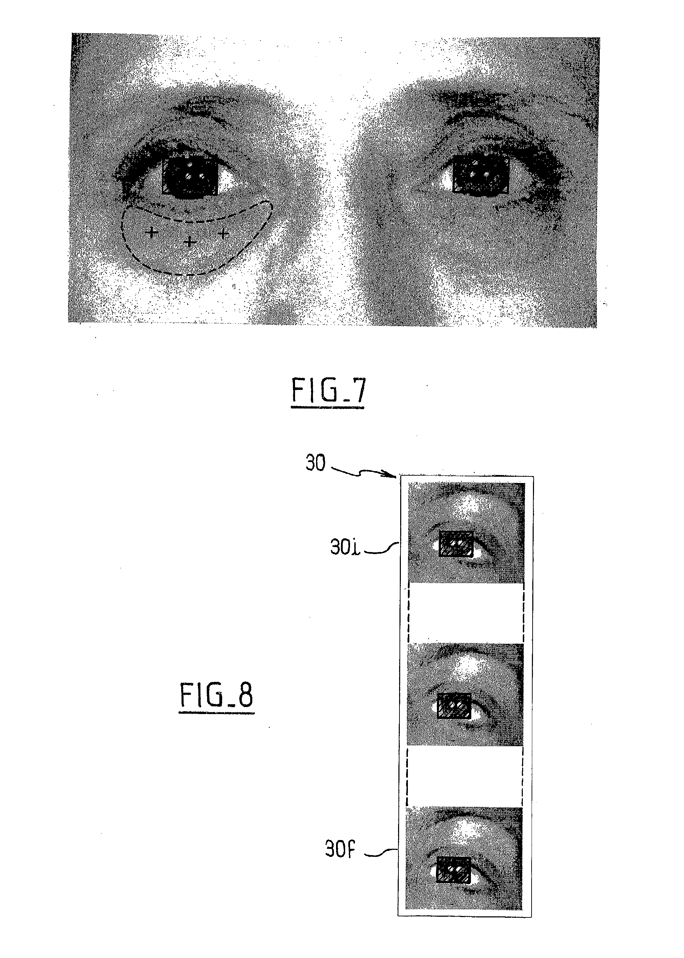

On a matérialisé par un trait discontinu sur la figure 5 un exemple de localisation de cernes ethniques, sur la figure 6 un exemple de localisation de cernes vasculaires et sur la figure 7 un exemple de localisation de faux cernes.

Les données colorimétriques, de relief et/ou concernant les propriétés mécaniques peuvent être comparées à des données prédéterminées, par exemple des données stockées dans une mémoire, afin de déterminer le type de cernes.

On peut par exemple effectuer, de manière automatique ou non, une comparaison entre une image acquise au moyen d'une caméra et une séquence d'images au cours de laquelle au moins une caractéristique de la typologie de la région autour de l'oeil varie, par exemple selon une progression continue, comme illustré sur la figure 8. Sur cette figure, on a représenté une séquence d'images 30 comportant une image de départ 30i et une image d'arrivée 30f, une telle séquence étant générée par exemple au moyen d'un programme de morphage. L'image qui est acquise de la région du contour de l'oeil et qui sert de référence peut être par exemple affichée sur un écran simultanément avec une image de la séquence, de manière à permettre à un opérateur ou à une personne effectuant un auto-diagnostic de sélectionner l'image de la séquence qui à son avis est la plus proche de l'image de référence. La comparaison entre l'image de référence et les images de la séquence peut également être effectuée sans affichage des images de la séquence, au moyen d'un moteur de reconnaissance d'images, par exemple. Dans ce cas, la délimitation automatique du contour des cernes par analyse de la couleur en chaque point de l'image peut s'avérer particulièrement utile. Un atlas comportant plusieurs images représentant différents types de cernes ou de poches peut encore être utilisé.

L'acquisition du relief de la région autour de l'oeil permet, éventuellement combinée à l'acquisition des caractéristiques mécaniques, de déterminer le type de poche.

L'acquisition du relief peut se faire en plusieurs points, par exemple ceux matérialisés par des croix sur la figure 7, afin de déterminer si la surface de la peau est généralement concave ou convexe, ce qui est représentatif d'une poche creuse ou pleine, respectivement.

Les poches creuses sont essentiellement dues à des causes morphoanatomiques, à l'affinement ou au relâchement des tissus tandis que les poches pleines ont une origine congénitale ou peuvent avoir une origine physiologique, par exemple inflammatoire.

On a répertorié dans le tableau II ci-dessous différents types de poches.

**TABLEAU II**

| **Types poches** | **Caractéristiques mécaniques** | **Localisation** | **Origine** | **Evolution** |
|---|---|---|---|---|
| Creuses | Peau distendue | Inférieure et supérieure | Relâchement des tissus avec l'age | Non réversible |
| Pleines non réversibles | Non spécifiques | Inférieure | Protrusion des loges graisseuses | Non réversible |
| Pleines en partie réversibles | Non spécifiques | Supérieure | Ptose | Plus ou moins réversible |
| Pleines réversibles | Peau tendue | Inférieure et supérieure | Gonflement lymphoedemateux | Réversible |

La détermination du type de poche peut s'effectuer éventuellement au moyen d'un programme de morphage, à l'instar des cernes.

Les données concernant la couleur, les propriétés mécaniques, le relief peuvent être complétées par l'acquisition de données résultant d'un questionnaire.

Un tel questionnaire peut être par exemple affiché à l'écran d'un ordinateur et comporter des questions relatives à l'évolution des cernes ou des poches au cours d'une journée et à l'existence de facteurs aggravants ou minimisant tels qu'un traitement médical, un état psychique particulier, l'exposition à certains environnements, ou le port de lunettes de soleil lors d'une exposition au soleil, par exemple. Le questionnaire peut notamment permettre d'acquérir des données sur la présence ou non de cernes ou poches au réveil ou le soir, et plus généralement sur le caractère réversible ou non des cernes ou des poches, au dire de la personne examinée.

Eventuellement, une substance peut être administrée afin de provoquer des pleurs par exemple. Il peut s'agir par exemple d'exposer l'oeil aux substances dégagées par une pelure d'oignon. En comparant l'aspect de la région de l'oeil avant et après l'exposition à la pelure d'oignon, on peut déterminer plus facilement certaines caractéristiques, par exemple mesurer le gonflement des poches.

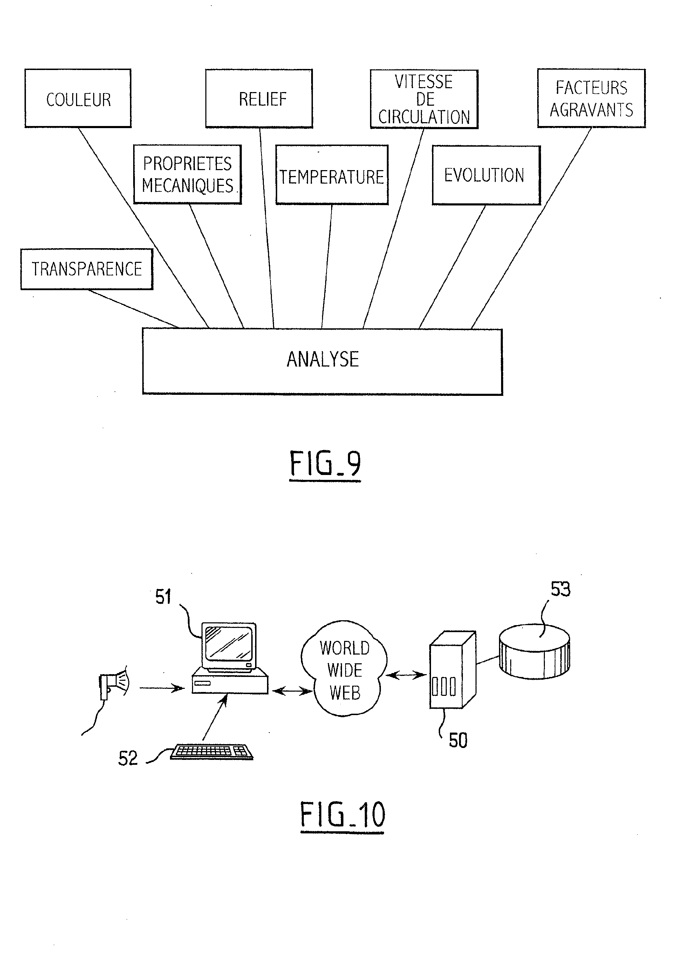

Les différentes données peuvent être analysées, comme illustré sur la figure 9, de manière à permettre la classification des cernes et des poches dans l'une des différentes catégories précitées.

En fonction des résultats de l'analyse, un traitement cosmétique, pharmacologique ou chirurgical peut être proposé.

Dans le cas de cernes ethniques, une dépigmentation peut être proposée par exemple et dans le cas de faux cernes un traitement chirurgical d'aplanissement des poches peut être effectué. Les poches creuses pourront être comblées et les poches pleines dues à la protrusion des loges graisseuses pourront être traitées par ablation partielle de la graisse. Les poches pleines dues à la ptose pourront être traitées par un lifting, par l'injection ou l'application de myorelaxants ou de myostimulants sur les muscles abaisseurs ou releveurs ou des fibres musculaires isolées.

Les résultats d'une acquisition de données colorimétriques, de relief et/ou de propriétés mécaniques, par exemple au moyen de l'appareil 10 précédemment décrit, ainsi éventuellement que d'autres informations entrées par exemple au moyen d'un clavier 52 relié à un ordinateur personnel 51, peuvent être transmis à distance, par exemple par l'intermédiaire du réseau Internet, à un serveur 50 comportant ou relié à une base de données 53, comme illustré à la figure 10.

Le serveur 50 peut être programmé pour effectuer, au vu des données transmises, un diagnostic et préconiser un soin ou un maquillage et des conseils.

Le serveur 50 peut également être agencé pour permettre de collecter les différentes données afin de constituer par exemple une banque de données multi vectorielle, dans laquelle chaque vecteur est spécifique à un individu et a pour composantes, parmi d'autres, le type de poches et le type de cernes de l'individu en question.

Ainsi, on peut par exemple déterminer en fonction d'une zone géographique les types de poches et de cernes prédominants et commercialiser dans cette zone géographique des produits adaptés.

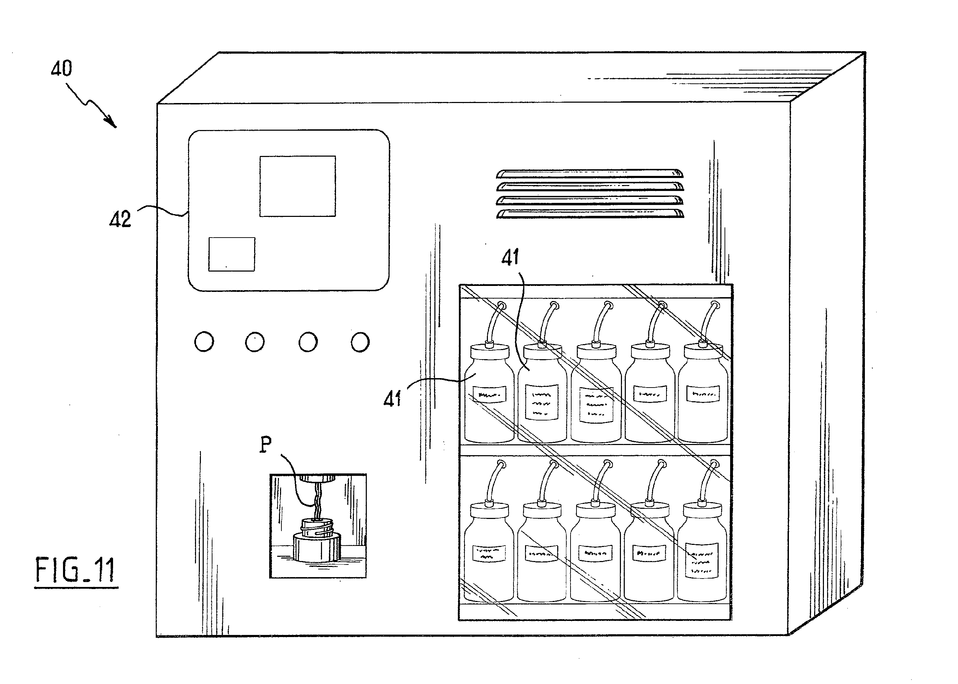

Les moyens d'acquisition de données concernant la couleur, la transparence, le relief et/ou les propriétés mécaniques de la région autour de l'oeil peuvent être reliés à un dispositif 40 représenté schématiquement à la figure 11, permettant de préparer en fonction du résultat de l'évaluation un produit cosmétique ou de traitement P, à partir d'une pluralité de récipients 41 contenant différents ingrédients.

Le dispositif 40 peut comporter par exemple un écran 42, notamment un écran tactile, lequel permet à un utilisateur d'entrer des réponses à un questionnaire.

Le dispositif 40 peut être présent dans un institut ou sur un point de vente, par exemple, et peut permettre de délivrer un produit de maquillage et/ou de soins personnalisé, correspondant au mieux aux types de cernes et de poches d'une personne précédemment examinée.

## Revendications

1. Procédé pour permettre d'évaluer la typologie de la région s'étendant autour de l'oeil humain, **caractérisé par le fait qu'**il comporte les étapes suivantes :
- permettre d'acquérir au moins une donnée d'au moins une zone de ladite région, associée à au moins un pigment présent dans le circuit sanguin et/ou à au moins un pigment externe au circuit sanguin,
- permettre d'acquérir au moins une donnée concernant le relief d'au moins une zone de ladite région et/ou au moins une donnée concernant au moins une propriété mécanique d'au moins une zone de ladite région,
- délivrer sur la base au moins des données précédemment acquises au moins une information concernant la typologie de ladite région.

2. Procédé selon la revendication 1, **caractérisé par le fait que** l'acquisition d'au moins une donnée associée à au moins un pigment présent dans le circuit sanguin et/ou à au moins un pigment externe au circuit sanguin comporte l'acquisition d'au moins une donnée concernant la couleur d'au moins une zone de ladite région.

3. Procédé selon l'une des revendications précédentes, **caractérisé par le fait qu'**il comporte en outre l'étape suivante :
- acquérir au moins une donnée relative aux circonstances d'apparition et/ou à l'évolution dans le temps d'au moins une caractéristique de ladite région.

4. Procédé selon l'une quelconque des revendications précédentes, **caractérisé par le fait qu'**il peut comporter l'étape consistant à déterminer la transparence de la peau d'au moins une zone de ladite région.

5. Procédé selon l'une quelconque des revendications précédentes, **caractérisé par le fait qu'**il comporte les étapes suivantes :
- acquérir au moins une donnée concernant la couleur d'au moins une zone de ladite région,
- acquérir au moins une donnée concernant le relief d'au moins une zone de ladite région,
- acquérir au moins une donnée concernant au moins une propriété mécanique d'au moins une zone de ladite région,
- délivrer sur la base au moins des données précédemment acquises au moins une information concernant la typologie de ladite région.

6. Procédé selon l'une quelconque des revendications précédentes, **caractérisé par le fait que** les données acquises sont comparées à des données prédéterminées et **par le fait qu'**au moins une information concernant la typologie de ladite région est délivrée au vu du résultat de cette comparaison.

7. Procédé selon l'une quelconque des revendications précédentes, **caractérisé par le fait que** l'on utilise pour acquérir au moins une donnée concernant la couleur d'au moins une zone de ladite région au moins l'un des éléments de la liste suivante : une caméra vidéo, un scanner, un appareil photo numérique, un colorimètre, un spectrocolorimètre, un nuancier, un SCIASCOPE™, un MEXAMETRE™, une caméra thermique.

8. Procédé selon l'une quelconque des revendications précédentes, **caractérisé par le fait que** l'on éclaire ladite région, lors de l'acquisition de ladite au moins une donnée concernant la couleur d'au moins une zone de ladite région, avec une lumière visible.

9. Procédé selon l'une quelconque des revendications 1 à 7, **caractérisé par le fait que** l'on éclaire ladite région, lors de l'acquisition de ladite au moins une donnée concernant la couleur d'au moins une zone de ladite région, avec une lumière ultraviolette.

10. Procédé selon l'une quelconque des revendications 1 à 7, **caractérisé par le fait que** l'on éclaire ladite région, lors de l'acquisition de ladite au moins une donnée concernant la couleur d'au moins une zone de ladite région, avec une lumière infrarouge.

11. Procédé selon l'une quelconque des revendications précédentes, **caractérisé par le fait que** l'on acquiert des données concernant la couleur d'au moins une zone de ladite région en éclairant successivement ladite zone avec des éclairages différents.

12. Procédé selon l'une quelconque des revendications précédentes, **caractérisé par le fait que** l'on acquiert au moins une donnée concernant le relief d'au moins une zone de ladite région au moyen d'un élément choisi dans la liste suivante : système à projection de franges, système à vision stéréoscopique, système d'imagerie à ultrasons, lampe à fente.

13. Procédé selon l'une quelconque des revendications précédentes,
**caractérisé par le fait que** l'on acquiert au moins une donnée concernant les propriétés mécaniques d'au moins une zone de ladite région au moyen d'un élément choisi dans la liste suivante : palpeur mécanique, dispositif à pincement, dispositif à torsion, dispositif à aspiration, dispositif à projection de liquide ou de gaz, tonomètre, dispositif pléthysmographique.

14. Procédé selon l'une quelconque des revendications précédentes, **caractérisé par le fait que** :
- l'on effectue dans un premier intervalle de temps au moins une première acquisition d'au moins une donnée concernant la couleur d'au moins une zone de ladite région et/ou d'au moins une donnée concernant le relief d'au moins une zone de ladite région et/ou d'au moins une donnée concernant au moins une propriété mécanique d'au moins une zone de ladite région,
- l'on procède dans un deuxième intervalle de temps succédant au premier à au moins une deuxième acquisition d'au moins une donnée concernant la couleur d'au moins une zone de ladite région et/ou d'au moins une donnée concernant le relief d'au moins une zone de ladite région et/ou d'au moins une donnée concernant au moins une propriété mécanique d'au moins une zone de ladite région,
- l'on compare les résultats des première et deuxième acquisitions, et
- l'on tire des résultats de la comparaison au moins une information de nature non médicale concernant la typologie de ladite région.

15. Procédé selon l'une quelconque des revendications précédentes, **caractérisé par le fait que** ladite au moins une information concernant la typologie de ladite région comporte une information de nature non médicale concernant les cernes.

16. Procédé selon la revendication précédente, **caractérisé par le fait que** ladite au moins une information concernant la typologie de ladite région comporte une information de nature non médicale permettant de classer les cernes dans une catégorie parmi un ensemble comportant plusieurs catégories.

17. Procédé selon la revendication précédente, **caractérisé par le fait que** ledit ensemble comporte au moins les cernes suivants : cernes ethniques, faux cernes, cernes vasculaires.

18. Procédé selon l'une quelconque des revendications précédentes, **caractérisé par le fait que** ladite au moins une information concernant la typologie de ladite région comporte une information de nature non médicale concernant les poches.

19. Procédé selon la revendication précédente, **caractérisé par le fait que** ladite au moins une information concernant la typologie de ladite région comporte une information de nature non médicale permettant de classer les poches dans une catégorie parmi un ensemble de plusieurs catégories, notamment un ensemble comportant au moins les catégories suivantes : poches creuses dues au relâchement des tissus, poches pleines dues à la protrusion des loges graisseuses, poches pleines dues à la ptose de certains tissus, poches pleines dues au gonflement lymphoedémateux.

20. Procédé selon l'une quelconque des revendications précédentes, **caractérisé par le fait que** ladite au moins une information concernant la typologie de ladite région comporte une information concernant les rides du coin de l'oeil.

21. Procédé pour évaluer l'incidence sur l'aspect de la région autour de l'oeil de l'application d'un produit cosmétique ou pour déterminer l'efficacité d'un traitement cosmétique, dans lequel :
- on procède à une première acquisition de données associées à au moins un pigment intérieur et/ou extérieur au circuit sanguin, notamment la couleur, et de données concernant le relief et/ou au moins une propriété mécanique d'au moins une zone de la région autour de l'oeil avant l'application d'un produit cosmétique et/ou un traitement,
- on applique un produit cosmétique ou on effectue un traitement cosmétique,
- on procède ensuite à une deuxième acquisition de données associées audit au moins un pigment intérieur et/ou extérieur au circuit sanguin, notamment la couleur, et de données concernant le relief et/ou au moins une propriété mécanique d'au moins une zone de la région autour de l'oeil,
- on détermine en comparant les résultats des première et deuxième acquisitions l'incidence sur l'aspect de la région autour de l'oeil de l'application du produit cosmétique ou du traitement.

22. Dispositif d'acquisition de données concernant la typologie de la région s'étendant autour de l'oeil humain, **caractérisé par le fait qu'**il comporte des moyens d'acquisition (11, 12, 13 ; 21,23) permettant d'acquérir :
- au moins une donnée associée à au moins un pigment présent dans le circuit sanguin et/ou à un pigment externe au circuit sanguin, notamment une donnée concernant la couleur d'au moins une zone de ladite région,
- et permettant d'acquérir également,
- au moins une donnée concernant le relief d'au moins une zone de ladite région et/ou,
- au moins une donnée concernant au moins une propriété mécanique d'au moins une zone de ladite région.

23. Dispositif selon la revendication précédente, **caractérisé par le fait qu'**il comporte en outre des moyens de traitement (15; 51) pour comparer les données acquises à des données mémorisées et générer au moins une information concernant la typologie de ladite région en fonction du résultat de cette comparaison.

24. Dispositif selon l'une des revendications 22 et 23, **caractérisé par le fait qu'**il est agencé pour permettre l'acquisition de données relatives à l'évolution d'au moins une caractéristique de ladite région dans le temps.

25. Dispositif selon l'une quelconque des revendications 22 à 24, **caractérisé par le fait qu'**il comporte des moyens d'acquisition colorimétriques comprenant au moins l'un des éléments de la liste suivante : une caméra vidéo, un scanner, un appareil photo numérique, un colorimètre, un spectrocolorimètre, un nuancier, un SCIASCOPE™, un MEXAMETRE™.

26. Dispositif selon l'une quelconque des revendications 22 à 25, **caractérisé par le fait qu'**il comporte des moyens d'éclairage (12) permettant d'éclairer ladite région avec une lumière visible, ultraviolette ou infrarouge.

27. Dispositif selon l'une quelconque des revendications 22 à 26, **caractérisé par le fait qu'**il comporte des moyens (11) permettant d'effectuer des acquisitions colorimétriques sous des éclairages différents.

28. Dispositif selon l'une quelconque des revendications 22 à 27, **caractérisé par le fait qu'**il comporte des moyens (13) d'acquisition du relief comportant au moins un élément choisi dans la liste suivante : système à projection de franges, système à vision stéréoscopique, système d'imagerie à ultrasons, lampe à fente.

29. Dispositif selon l'une quelconque des revendications 22 à 28, **caractérisé par le fait qu'**il comporte des moyens (14) d'acquisition de propriétés mécaniques comportant au moins un élément choisi dans la liste suivante : palpeur mécanique, dispositif à pincement, dispositif à torsion, dispositif à aspiration, dispositif à projection de liquide ou de gaz, dispositif tonométrique, dispositif pléthysmographique.

30. Dispositif selon l'une quelconque des revendications 22 à 29, **caractérisé par le fait qu'**il comporte des moyens de stockage (15 ; 51) permettant de stocker des données acquises à différents instants.

31. Dispositif selon l'une quelconque des revendications 22 à 30, **caractérisé par le fait qu'**il comporte un cache (18) agencé pour s'appliquer par la totalité de son pourtour contre une région de contour de l'oeil.

32. Procédé pour diagnostiquer au moins un état de la région du contour de l'oeil, comportant les étapes suivantes :
- permettre d'acquérir au moins une donnée d'au moins une zone de ladite région, associée à au moins un pigment présent dans le circuit sanguin et/ou à au moins un pigment externe au circuit sanguin,
- permettre d'acquérir au moins une donnée concernant le relief d'au moins une zone de ladite région et/ou au moins une donnée concernant au moins une propriété mécanique d'au moins une zone de ladite région,
- délivrer un diagnostic non médical sur la base au moins des données précédemment acquises.

33. Procédé pour prescrire un traitement cosmétique, comportant les étapes suivantes :
- permettre d'acquérir au moins une donnée d'au moins une zone de ladite région, associée à au moins un pigment présent dans le circuit sanguin et/ou à au moins un pigment externe au circuit sanguin,
- permettre d'acquérir au moins une donnée concernant le relief d'au moins une zone de ladite région et/ou au moins une donnée concernant au moins une propriété mécanique d'au moins une zone de ladite région,
- prescrire un traitement sur la base au moins des données précédemment acquises.

34. Kit permettant à un individu de s'auto-diagnostiquer, comportant
- des moyens permettant d'acquérir au moins une donnée d'au moins une zone de ladite région, associée à au moins un pigment présent dans le circuit sanguin et/ou à au moins un pigment externe au circuit sanguin,
- des moyens permettant d'acquérir au moins une donnée concernant le relief d'au moins une zone de ladite région et/ou au moins une donnée concernant au moins une propriété mécanique d'au moins une zone de ladite région,
- des moyens permettant de délivrer un diagnostic, notamment un diagnostic non médical, sur la base au moins des données précédemment acquises.

35. Procédé de fabrication d'un produit, notamment cosmétique, comportant les étapes suivantes :
- permettre d'acquérir au moins une donnée d'au moins une zone de ladite région, associées à au moins un pigment présent dans le circuit sanguin et/ou à au moins un pigment externe au circuit sanguin,
- permettre d'acquérir au moins une donnée concernant le relief d'au moins une zone de ladite région et/ou au moins une donnée concernant des propriétés mécaniques d'au moins une zone de ladite région,
- fabriquer un produit, notamment cosmétique, au moins sur la base des données précédemment acquises.

36. Procédé pour générer un panel d'utilisateurs potentiels d'un produit, notamment cosmétique, comportant les étapes suivantes :
- pour chaque individu d'un groupe, acquérir :
- au moins une donnée d'au moins une zone de ladite région, associée à au moins un pigment présent dans le circuit sanguin et/ou à au moins un pigment externe au circuit sanguin,
- au moins une donnée concernant le relief d'au moins une zone de ladite région et/ou au moins une donnée concernant au moins une propriété mécanique d'au moins une zone de ladite région,
- sélectionner parmi les individus de ce groupe ceux dont les données précédemment acquises répondent à au moins un critère prédéterminé.

37. Procédé selon la revendication 36, **caractérisé par le fait qu'**il comporte en outre les étapes suivantes :
i) générer un panel,
ii) fabriquer un produit, notamment un produit cosmétique,
iii) appliquer ou administrer le produit à des individus du panel,
iv) effectuer une nouvelle évaluation,
v) comparer les résultats de la nouvelle évaluation avec ceux de la ou des évaluations précédentes, en vue de déterminer l'efficacité du produit,
vi) si l'efficacité du produit est jugée insuffisante, modifier la formulation du produit et/ou sa posologie, et recommencer les étapes iii), iv) et v) jusqu'à l'obtention d'une efficacité jugée suffisante.

38. Procédé pour déterminer l'efficacité d'un traitement cosmétique, comportant les étapes suivantes :
- effectuer une première évaluation de la région du contour de l'oeil au moyen du procédé tel que défini dans l'une quelconque des revendications 1 à 20,
- effectuer un traitement,
- effectuer une nouvelle évaluation
- comparer les résultats des évaluations et en tirer une information utile sur l'efficacité du traitement.
